# EUROPEAN PATENT APPLICATION

(11) **EP 4 725 484 A2**
(43) Date of publication of application: **15.04.2026**
(21) Application number: 26159633.2
(22) Date of filing: 23.12.2022
(51) Int. Cl.: A61K 31/454

(54) **CRYSTALLINE FORM OF GLP-1 RECEPTOR AGONIST AND PREPARATION METHOD THEREFOR**

(30) Priority: 23.12.2021 CN 202111586541
(62) Divisional of application: 22910190.2
(71) Applicant: Jiangsu Hengrui Pharmaceuticals Co., Ltd., Lianyungang, Jiangsu 222047 (CN)
(72) Inventor: XU, Gujun, Lianyungang, 222047 (CN); LU, Weidong, Shanghai, 200245 (CN); YANG, Junran, Lianyungang, 222047 (CN); DU, Zhenxing, Lianyungang, 222047 (CN); SHAO, Qiyun, Shanghai, 200245 (CN)
(74) Representative: J A Kemp LLP

(57) **Abstract**

A crystalline form of a GLP-1 receptor agonist and a preparation method therefor. The agonist is a compound 2-((4-((S)-3-(4-chloro-2-fluorophenyl)-2,3-dihydrobenzo[b][1,4]dioxane-5-yl)piperidin-1-yl)methyl)-1-(((S)-oxe-tan-2-yl)methyl)-1H-benzo[d]imidazole-6-carboxylic acid.

## Description

The present application claims the priority of China Patent Application 2021115865413 filed on December 23, 2021. This Chinese patent application is incorporated herein by reference in its entirety.

### TECHNICAL FIELD

The present disclosure belongs to the field of medicines, and relates to a pharmaceutically acceptable salt and a crystalline form of a GLP-1 receptor agonist and a preparation method therefor.

### BACKGROUND

Glucagon-like peptide-1 (GLP-1) is an incretin hormone secreted by L-cells in the lower digestive tract. GLP-1 plays a corresponding role by binding to its ubiquitous specific receptor. Organs in which GLP-1 receptor is now clearly present include islet cells, gastrointestinal, pulmonary, brain, kidney, hypothalamus and cardiovascular systems, and GLP-1 receptor may also be present in liver, adipose tissues and skeletal muscle. GLP-1 not only acts on β cells to promote insulin secretion, but also acts on α cells to inhibit glucagon secretion. There is generally no significant difference in serum GLP-1 levels in patients with normal glucose tolerance, impaired glucose tolerance, and type II diabetes. However, there is a deficiency of the response of β cells to GLP-1 after eating, and under certain conditions, the response is significantly enhanced after continuous infusion of GLP-1. Since the duration of action of human GLP-1 is very short (t1/2 < 1.5 minutes via intravenous injection), human GLP-1 is not suitable for clinical treatment of diabetes.

Peptidic GLP-1 receptor agonists (e.g., liraglutide and exenatide) have effects on improving blood glucose level in type II diabetic patients by lowering fasting and postprandial glucose. However, since the peptidic GLP-1 has poor oral bioavailability and is inconvenient to take, small molecule agonists of GLP-1 receptors with good oral bioavailability are highly desirable.

2-((4-((,S)-3-(4-chloro-2-fluorophenyl)-2,3-dihydrobenzo[b][1,4]dioxan-5-yl)piperidin-1-yl)methyl)-1-(((*S*)-oxetan-2-yl)methyl)-1*H*-benzo[*d*]imidazole-6-carboxylic acid is a novel GLP-1 receptor agonist (the structural formula is as follows, PCT/CN2021/115915),

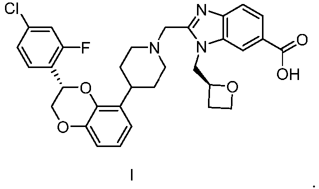

A crystal form acting as a pharmaceutical active ingredient often affects the chemical stability of a drug. Different crystallization conditions and storage conditions may lead to changes in the crystal structure of a compound, sometimes accompanied with the production of other forms of crystal forms. In general, an amorphous drug product has an irregular crystal structure, which often leads to other defects, such as poor product stability, finer crystallization, difficulty in filtration, easy to agglomerate, and poor fluidity. Polymorphism of drugs have different requirements for product storage, production and scale up. Therefore, it is necessary to deeply study the crystal forms of the above-mentioned compounds and improve the various properties thereof.

### CONTENT OF THE PRESENT INVENTION

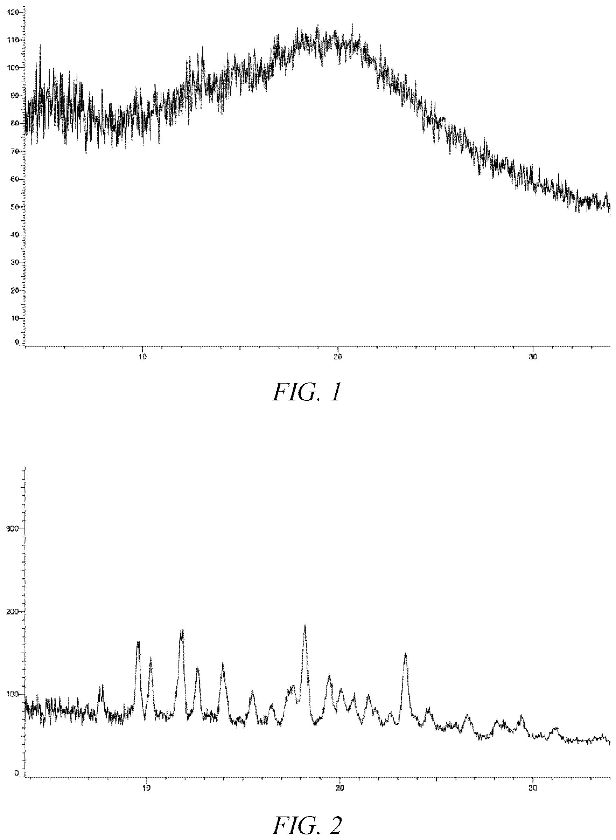

The present disclosure provides an amorphous form of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, and an X-ray powder diffraction pattern expressed with diffraction angle 2*θ* has no obvious characteristic peaks. In some embodiments, the amorphous form has an X-ray powder diffraction pattern expressed with diffraction angle 2*θ* in which there are no obvious characteristic peaks in the range of 0 ° to 40 °. The X-ray powder diffraction pattern is as shown in FIG. 1.

The present disclosure further provides a method for preparing the amorphous form of the compound mentioned above, which comprises: a step of mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with water, and stirring the mixture.

In certain embodiments, the volume (µL) of the solvent 1 used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

In another aspect, the present disclosure further provides crystal form A of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[d]imidazole-6-carboxylic acid.

In some embodiments, the crystal form A of the compound has an X-ray powder diffraction pattern expressed with diffraction angle 2*θ* in which there are characteristic peaks at 9.587, 10.216, 11.812, 18.204, and 23.404.

In some embodiments, the crystal form A of the compound has characteristic peaks at 9.587, 10.216, 11.812, 12.645, 13.956, 15.488, 17.541, 18.204, 19.462, and 23.404.

In some embodiments, the crystal form A of the compound has characteristic peaks at 7.654, 9.587, 10.216, 11.812, 12.645, 13.956, 15.488, 16.503, 17.541, 18.204, 19.462, 20.041, 20.697, 21.477, 21.812, 22.615, 23.404, 24.533, 26.618, 28.168, 29.406, and 31.044.

In some embodiments, the crystal form A of the compound has an X-ray powder diffraction pattern expressed with diffraction angle 2*θ* as shown in FIG. 2.

The present disclosure further provides a method for preparing the crystal form A of the compound mentioned above, which comprises:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (1), and dissolving the mixture by means of stirring or heating,
(b) crystallization, wherein the solvent (1) is selected from one or more of n-propanol, nitromethane, tetrahydrofuran, isopropanol, isopropyl acetate, methyl tert-butyl ether, acetonitrile, ethyl acetate, and n-hexane.

In some embodiments, the crystallization mode of the crystal form A of the compound is stirring crystallization, static crystallization, cooling crystallization, cooling crystallization with stirring, or volatilization crystallization.

In some embodiments, the method for preparing the crystal form A of the compound mentioned above comprises mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with tetrahydrofuran, and dissolving the mixture by means of stirring or heating, and (b) volatilization crystallization.

In certain embodiments, the volume (µL) of the solvent (1) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

In some embodiments, the preparation method described in the present disclosure further comprises a step of filtration, washing, or drying.

The present disclosure further provides crystal form B1 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form B1 of the compound has an X-ray powder diffraction pattern expressed with diffraction angle 2*θ* in which there are characteristic peaks at 8.135, 8.915, 11.259, 11.508, 19.024, and 25.271.

In some embodiments, the crystal form B1 of the compound has characteristic peaks at 8.135, 8.915, 10.507, 11.259, 11.508, 12.223, 16.751, 19.024, 22.736, and 25.271.

In some embodiments, the crystal form B1 of the compound has characteristic peaks at 8.135, 8.915, 10.507, 11.259, 11.508, 12.223, 13.632, 15.055, 16.751, 17.836, 19.024, 20.541, 22.205, 22.736, 25.271, and 26.849.

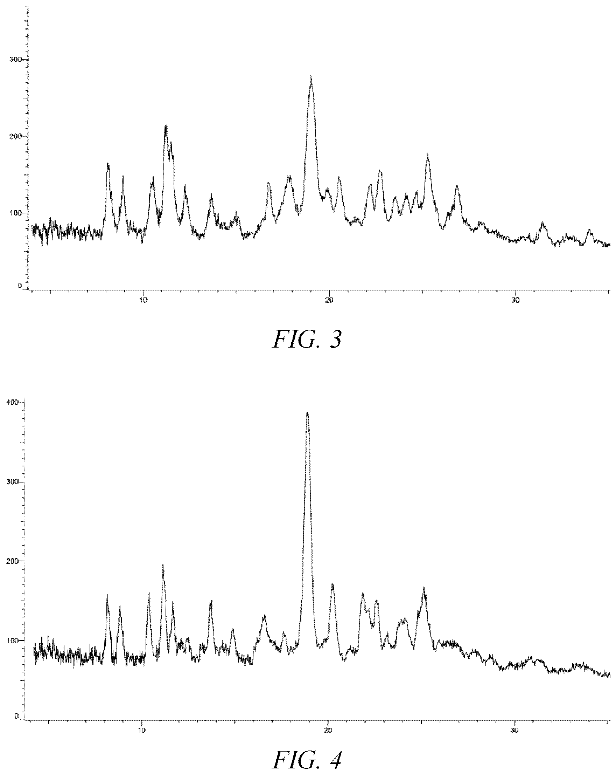

In some embodiments, the crystal form B1 of the compound has an X-ray powder diffraction pattern expressed with diffraction angle 2*θ* as shown in FIG. 3.

The present disclosure further provides a method for preparing the crystal form B1 of the compound mentioned above, and the method is selected from any one of the following methods:
method I:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (2), and dissolving the mixture by means of stirring or heating, wherein the solvent is selected from one or more of isopropanol, isopentanol, 1,2-dichloroethane, acetone, isopropyl acetate, propylene glycol methyl ether, and p-xylene,
   (b) crystallization;
or method II:
   (a) mixing compound 2-((4-((*S*)-3-(4-chloro-2-fluorophenyl)-2,3-dihydrobenzo [*b*][1,4]dioxan-5-yl)piperidin-1-yl)methyl)-1-(((S)-oxetan-2-yl)methyl)-1*H*-benzo[*d*]imidazole-6-carboxylic acid with a solvent (3), and dissolving the mixture by means of stirring or heating, wherein the solvent (3) is tetrahydrofuran,
   (b) adding a solvent (4) for crystallization, wherein the solvent (4) is water;
or method III:
   (a) mixing compound 2-((4-((*S*)-3-(4-chloro-2-fluorophenyl)-2,3-dihydrobenzo [*b*][1,4]dioxan-5-yl)piperidin-1-yl)methyl)-1-(((*S*)-oxetan-2-yl)methyl)-1*H*-benzo[*d*]imidazole-6-carboxylic acid with a solvent (5), wherein the solution (5) is selected from dioxane,
   (b) stirring and pulping.

In some embodiments, the crystallization mode of the crystal form B1 of the compound is stirring crystallization, static crystallization, cooling crystallization, cooling crystallization with stirring, or volatilization crystallization.

In some embodiments, the method for preparing the crystal form B1 of the compound mentioned above comprises mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with acetone, and dissolving the mixture by means of stirring or heating, and volatilization crystallization.

In certain embodiments, the volume (µL) of the solvents (2), (3), (4) and (5) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form B2 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form B2 of the compound has an X-ray powder diffraction pattern expressed with diffraction angle 2*θ* in which there are characteristic peaks at 8.182, 8.839, 10.401, 11.168, and 18.906.

In some embodiments, the crystal form B2 of the compound has characteristic peaks at 8.182, 8.839, 10.401, 11.168, 11.679, 13.714, 18.906, 20.245, 21.895, and 25.134.

In some embodiments, the crystal form B2 of the compound has characteristic peaks at 8.182, 8.839, 10.401, 11.168, 11.679, 13.714, 14.880, 16.592, 17.660, 18.906, 20.245, 21.895, 22.600, and 25.134.

In some embodiments, the crystal form B2 of the compound has an X-ray powder diffraction pattern expressed with diffraction angle 2*θ* as shown in FIG. 4.

The present disclosure further provides a method for preparing the crystal form B2 of the compound mentioned above, which comprises:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (6), and dissolving the mixture by means of stirring or heating, wherein the solvent (6) is selected from methyl isobutyl ketone,
(b) crystallization.

In some embodiments, the crystallization mode of the crystal form B2 of the compound is stirring crystallization, static crystallization, cooling crystallization, cooling crystallization with stirring, or volatilization crystallization.

In certain embodiments, the volume (µL) of the solvent (6) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form B3 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form B3 of the compound has an X-ray powder diffraction pattern expressed with diffraction angle 2*θ* in which there are characteristic peaks at 10.548, 11.496, 17.557, 19.135, 19.751, and 25.360.

In some embodiments, the crystal form B3 of the compound has characteristic peaks at 10.548, 11.269, 11.496, 17.557, 18.103, 19.135, 19.751, 20.605, 22.767, and 25.360.

In some embodiments, the crystal form B3 of the compound has characteristic peaks at 8.224, 8.976, 10.548, 11.269, 11.496, 12.264, 13.730, 14.829, 17.557, 18.103, 19.135, 19.751, 20.605, 22.767, 23.522, 24.738, 25.360, 26.556, and 26.893.

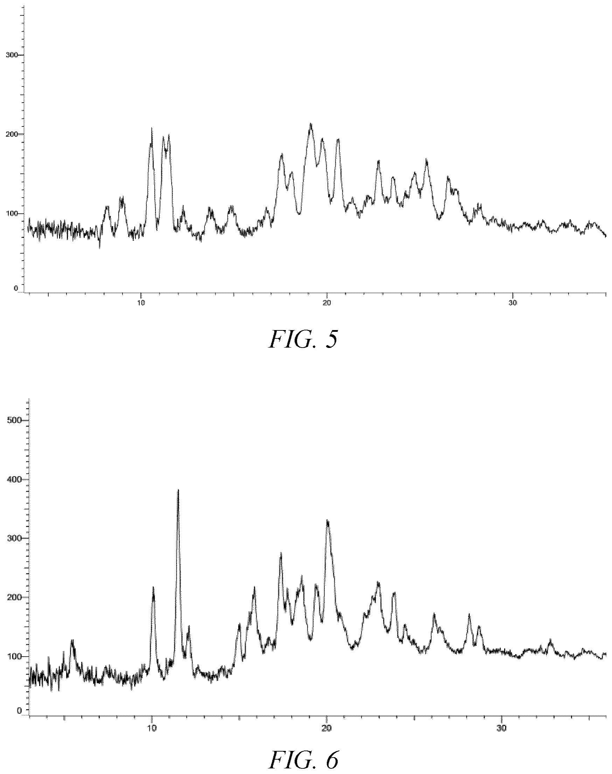

In some embodiments, the crystal form B3 of the compound has an X-ray powder diffraction pattern expressed with diffraction angle 2*θ* as shown in FIG. 5.

The present disclosure further provides a method for preparing the crystal form B3 of the compound mentioned above, which comprises:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (7), and dissolving the mixture by means of stirring or heating, wherein the solvent (7) is selected from one or more of ethyl acetate, dichloromethane, and 10% water/acetone,
(b) crystallization.

In some embodiments, the crystallization mode of the crystal form B3 of the compound is stirring crystallization, static crystallization, cooling crystallization, cooling crystallization with stirring, or volatilization crystallization.

In certain embodiments, the volume (µL) of the solvent (7) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form C of compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form C of the compound has an X-ray powder diffraction pattern expressed with diffraction angle 2*θ* in which there are characteristic peaks at 10.094, 11.511, 17.378, and 20.113.

In some embodiments, the crystal form C of the compound has characteristic peaks at 10.094, 11.511, 15.875, 17.378, 17.763, 18.573, 20.113, and 22.925.

In some embodiments, the crystal form C of the compound has characteristic peaks at 5.470, 10.094, 11.511, 12.138, 14.975, 15.875, 17.378, 17.763, 18.573, 19.413, 20.113, 22.925, 23.881, 26.177, and 28.163.

In some embodiments, the crystal form C of the compound has an X-ray powder diffraction pattern expressed with diffraction angle 2*θ* as shown in FIG. 6.

The present disclosure further provides a method for preparing the crystal form C of the compound mentioned above, and the method is selected from any one of the following methods:
method (I):
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (8), and dissolving the mixture by means of stirring or heating, wherein the solvent (8) is selected from toluene,
   (b) crystallization;
or method (II):
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((S)-oxetan-2-yl)methyl)-1*H-*benzo[*d*]imidazole-6-carboxylic acid with a solvent (9), wherein the solvent (9) is selected from isopropyl acetate,
   (b) stirring and pulping.

In some embodiments, the crystallization mode of the crystal form C of the compound is stirring crystallization, static crystallization, cooling crystallization, cooling crystallization with stirring, or volatilization crystallization.

In certain embodiments, the volume (µL) of the solvents (8) and (9) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form D of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form D of the compound has an X-ray powder diffraction pattern expressed with diffraction angle 2*θ* in which there are characteristic peaks at 10.940, 12.216, 18.344, 19.931, and 22.979.

In some embodiments, the crystal form D of the compound has characteristic peaks at 6.343, 10.940, 12.216, 17.695, 18.344, 18.973, 19.472, 19.931, 21.753, 22.979, and 24.685.

In some embodiments, the crystal form D of the compound has characteristic peaks at 6.343, 10.940, 12.216, 12.762, 14.684, 16.167, 16.510, 17.695, 18.344, 18.973, 19.472, 19.931, 21.753, 22.979, 24.306, 24.685, and 25.898.

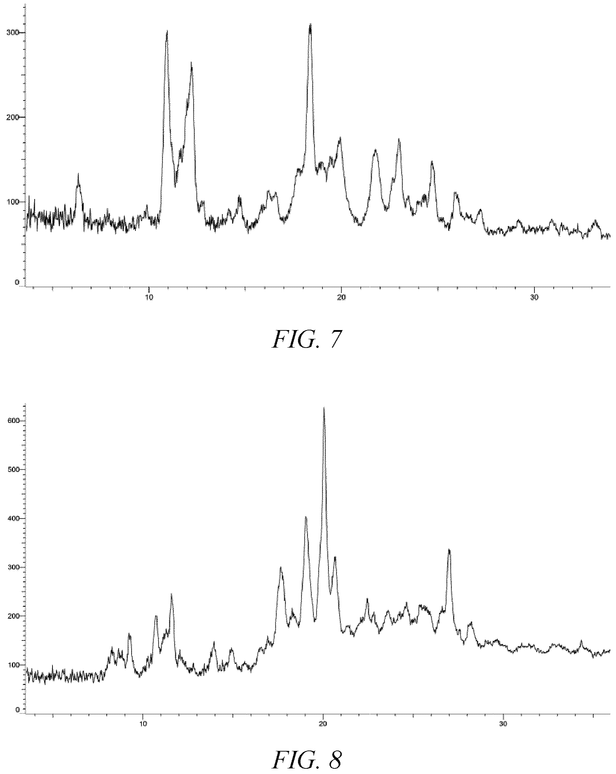

In some embodiments, the crystal form D of the compound has an X-ray powder diffraction pattern expressed with diffraction angle 2*θ* as shown in FIG. 7.

The present disclosure further provides a method for preparing the crystal form D of the compound mentioned above, and the method is selected from any one of the following methods:
method I:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (10), and dissolving the mixture by means of stirring or heating, wherein the solvent (10) is selected from 50% methanol/water and 50% acetonitrile/methanol,
   (b) crystallization;
or method II:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (11), and dissolving the mixture by means of stirring or heating, wherein the solvent (11) is selected from tetrahydrofuran or chloroform,
   (b) adding a solvent (12) for crystallization, wherein the solvent (12) is selected from methanol;
or method III:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (13), wherein the solvent (13) is selected from methanol,
   (b) stirring and pulping.

In some embodiments, the crystallization mode of the crystal form D of the compound is stirring crystallization, static crystallization, cooling crystallization, cooling crystallization with stirring, or volatilization crystallization.

In certain embodiments, the volume (µL) of the solvents (11), (12) and (13) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form E of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form E of the compound has an X-ray powder diffraction pattern expressed with diffraction angle 2*θ* in which there are characteristic peaks at 11.591, 17.645, 19.060, 20.066, 20.667, and 26.987.

In some embodiments, the crystal form E of the compound has characteristic peaks at 9.261, 10.735, 11.591, 13.946, 17.645, 18.291, 19.060, 20.066, 20.667, and 26.987.

In some embodiments, the crystal form E of the compound has characteristic peaks at 8.245, 8.738, 9.261, 10.735, 11.591, 12.056, 13.946, 14.925, 16.922, 17.645, 18.291, 19.060, 20.066, 20.667, 22.474, 24.608, and 26.987.

In some embodiments, the crystal form E of the compound has an X-ray powder diffraction pattern expressed with diffraction angle 2*θ* as shown in FIG. 8.

The present disclosure further provides a method for preparing the crystal form E of the compound mentioned above, and the method is selected from any one of the following methods:
method I:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (14), and dissolving the mixture by means of stirring or heating, wherein the solvent (14) is selected from acetonitrile,
   (b) crystallization;
or method II:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (15), and dissolving the mixture by means of stirring or heating, wherein the solvent (15) is selected from tetrahydrofuran,
   (b) adding a solvent (16) for crystallization, wherein the solvent (16) is selected from acetonitrile.

In some embodiments, the crystallization mode of the crystal form E of the compound is stirring crystallization, static crystallization, cooling crystallization, cooling crystallization with stirring, or volatilization crystallization.

In certain embodiments, the volume (µL) of the solvents (14), (15) and (16) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form F of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form F of the compound has an X-ray powder diffraction pattern expressed with diffraction angle 2*θ* in which there are characteristic peaks at 9.543, 19.405, and 22.153.

In some embodiments, the crystal form F of the compound has characteristic peaks at 9.543, 11.421, 14.557, 16.175, 17.886, 19.405, 22.153, and 25.821.

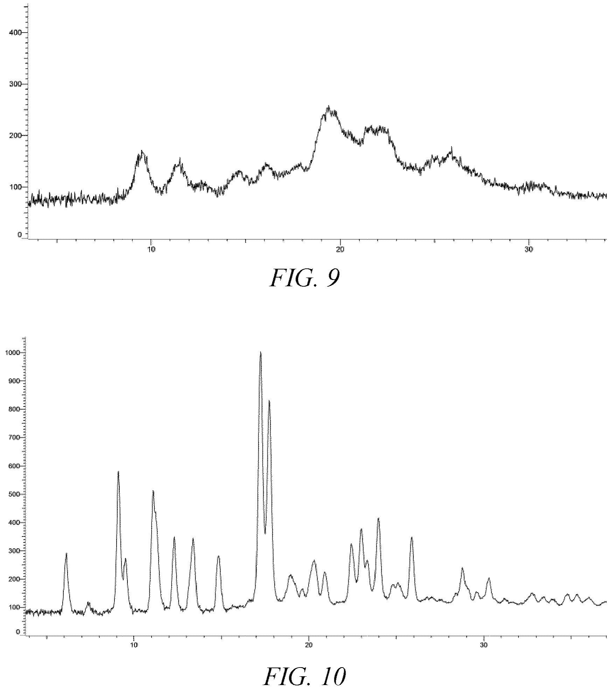

In some embodiments, the crystal form F of the compound has an X-ray powder diffraction pattern expressed with diffraction angle 2*θ* as shown in FIG. 9.

The present disclosure further provides a method for preparing the crystal form F of the compound mentioned above, and the method is selected from any one of the following methods:
method I:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (17), and dissolving the mixture by means of stirring or heating, wherein the solvent (17) is selected from dimethyl sulfoxide,
   (b) crystallization;
or method II:
   (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (18), and dissolving the mixture by means of stirring or heating, wherein the solvent (18) is selected from dimethyl sulfoxide,
   (b) adding a solvent (19) for crystallization, wherein the solvent (19) is selected from water.

In some embodiments, the crystallization mode of the crystal form F of the compound is stirring crystallization, static crystallization, cooling crystallization, cooling crystallization with stirring, or volatilization crystallization.

In certain embodiments, the volume (µL) of the solvents (17), (18) and (19) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

The present disclosure further provides crystal form G of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid.

In some embodiments, the crystal form G of the compound has an X-ray powder diffraction pattern expressed with diffraction angle 2*θ* in which there are characteristic peaks at 9.096, 11.107, 17.239, and 17.744.

In some embodiments, the crystal form G of the compound has characteristic peaks at 6.120, 9.096, 11.107, 12.302, 13.387, 17.239, 17.744, 22.984, 23.981, and 25.879.

In some embodiments, the crystal form G of the compound has characteristic peaks at 6.120, 9.096, 9.519, 11.107, 12.302, 13.387, 14.833, 17.239, 17.744, 20.302, 20.905, 22.416, 22.984, 23.342, 23.981, 25.879, and 28.791.

In some embodiments, the crystal form G of the compound has an X-ray powder diffraction pattern expressed with diffraction angle 2*θ* as shown in FIG. 10.

The present disclosure further provides a method for preparing the crystal form G of the compound mentioned above, which comprises:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent (20), and dissolving the mixture by means of stirring or heating, wherein the solvent (20) is selected from n-propanol,
(b) crystallization.

In some embodiments, the crystallization mode of the crystal form G of the compound is stirring crystallization, static crystallization, cooling crystallization, cooling crystallization with stirring, or volatilization crystallization.

In certain embodiments, the volume (µL) of the solvent (20) used in the present disclosure may be 1-200 times the mass (mg) of the compound mentioned above, and in non-limiting embodiments, may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, or values between any two of the numbers.

In addition, the method for preparing the above-mentioned crystal forms in the present disclosure further comprises one or more steps of filtration, washing, or drying.

The present disclosure further provides a pharmaceutical composition, which comprises the above-mentioned crystal form A, B1, B2, B3, C, D, E, F, or G, or crystal form A, B1, B2, B3, C, D, E, F, or G prepared by the above-mentioned method, and optionally pharmaceutical auxiliaries selected from pharmaceutically acceptable excipients.

The present disclosure further provides a method for preparing a pharmaceutical composition, the method comprising a step of mixing the above-mentioned crystal form A, B1, B2, B3, C, D, E, F, or G, or crystal form A, B1, B2, B3, C, D, E, F, or G prepared by the above-mentioned method with a pharmaceutically acceptable excipient.

The present disclosure further provides the use of the above-mentioned crystal form A, B1, B2, B3, C, D, E, F, or G, or crystal form A, B1, B2, B3, C, D, E, F, or G prepared by the above-mentioned method, or the above-mentioned composition in the preparation of a medicament for treating or preventing a disease associated with GLP-1 receptor.

The present disclosure further provides the use of the above-mentioned crystal form A, B1, B2, B3, C, D, E, F, or G, or crystal form A, B1, B2, B3, C, D, E, F, or G prepared by the above-mentioned method, or the above-mentioned composition in the preparation of a medicament for treating or preventing diabetes.

The "2*θ* or angle 2*θ*" mentioned in the present disclosure refers to a diffraction angle, and *θ* is a Bragg angle in ° or degrees; and the error range of 2θ for each characteristic peak is ± 0.2 (including the rounding of numbers with more than 1 decimal place), and may be -0.20, -0.19, -0.18, -0.17, -0.16, -0.15, -0.14, -0.13, -0.12, -0.11, -0.10, -0.09, -0.08, -0.07, -0.06, - 0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

According to the description of hygroscopic characteristics and the definition of hygroscopic weight gain in "9103 Guidelines for Hygroscopicity of Pharmaceuticals", Chinese Pharmacopoeia, Volume IV, 2015 edition, the following are defined:
deliquescent: absorbing enough water to form a liquid;
extremely hygroscopic: having a hygroscopic weight gain of not less than 15%;
hygroscopic: having a hygroscopic weight gain of less than 15% and not less than 2%;
slightly hygroscopic: having a hygroscopic weight gain of less than 2% and not less than 0.2%; and
no or almost no hygroscopicity: having a hygroscopic weight gain of less than 0.2%.

The "differential scanning calorimetry or DSC" mentioned in the present disclosure refers to measuring the temperature difference and heat flow difference between a sample and a reference during the process in which the sample is heated or maintained at a constant temperature, so as to characterize all physical and chemical changes related to thermal effects and obtain the phase change information of the sample.

In the present disclosure, the drying is generally at a temperature of 25 °C to 100 °C, preferably 40 °C to 70 °C, and may be either drying under atmospheric pressure or drying under reduced pressure with the pressure < -0.08 MPa.

The "excipient" mentioned in the present disclosure includes, but is not limited to, any adjuvant, carrier, glidant, sweetener, diluent, preservative, dye/colorant, flavoring agent, surfactant, wetting agent, dispersant, suspending agent, stabilizer, isotonic agent, or emulsifier that has been approved by the US Food and Drug Administration to be acceptable for human or livestock use.

The "pulping" mentioned in the present disclosure refers to a method of purification by means of the characteristic that a substance has poor solubility in a solvent while impurities have good solubility in the solvent. Pulping purification may result in discoloration, change the crystal form, or remove a small amount of impurities.

The starting raw materials used in the method for preparing the crystal forms in the present disclosure can be any form of compounds, and specific forms include, but are not limited to, amorphous form, any crystal form, hydrate, solvate, etc.

If the content of related substances is measured and calculated data, the numerical values in the present disclosure will inevitably have errors to some extent. Generally, ± 10% is within a reasonable error range. There is a certain degree of error variation in the context where it is used. The error variation does not exceed ±10%, and may be ±9%, ±8%, ±7%, ±6%, ±5%, ±4%, ±3%, ±2%, or ±1%, preferably ±5%.

The compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid (hereinafter referred to as Compound A) in the present disclosure is prepared by referring to a method in PCT/CN2021/115915, the relevant content of which is cited herein for illustration.

### BRIEF DESCRIPTION OF THE DRAWINGS

FIG. 1: an XRPD pattern of an amorphous form of Compound A prepared by Example 1.
FIG. 2: an XRPD pattern of crystal form A of Compound A prepared by Example 3.
FIG. 3: an XRPD pattern of crystal form B1 of Compound A prepared by Example 10.
FIG. 4: an XRPD pattern of crystal form B2 of Compound A prepared by Example 15.
FIG. 5: an XRPD pattern of crystal form B3 of Compound A prepared by Example 16.
FIG. 6: an XRPD pattern of crystal form C of Compound A prepared by Example 22.
FIG. 7: an XRPD pattern of crystal form D of Compound A prepared by Example 23.
FIG. 8: an XRPD pattern of crystal form E of Compound A prepared by Example 28.
FIG. 9: an XRPD pattern of crystal form F of Compound A prepared by Example 29.
FIG. 10: an XRPD pattern of crystal form G of Compound A prepared by Example 32.

### DETAILED DESCRIPTION OF THE PREFERRED EMBODIMENT

The present disclosure will be explained in more detail below in conjunction with examples or experimental examples. The examples or experimental examples in the present disclosure are only used to illustrate the technical solutions of the present disclosure and do not limit the essence and scope of the present disclosure.

Test conditions for instruments used in experiments in the present disclosure:
1. Differential Scanning Calorimeter (DSC)
   Instrument model: Mettler Toledo DSC 3+STARe System
   Purge gas: nitrogen nitrogen purging speed: 50 mL/min
   Heating rate: 10.0 °C/min
   Temperature range: 25-350 °C or 25-300 °C
2. X-ray Powder Diffraction (XRPD)
   Instrument model: BRUKER D8 Discover X-ray powder diffractometer
   Ray: monochromatic Cu-Kα ray (λ = 1.5406)
   Scanning mode: θ /2θ, scanning range (2θ range): 3 ° to 50 °
   Voltage: 40 kV, current: 40 mA
3. Thermogravimetric Analysis (TGA)
   Instrument model: Mettler Toledo TGA2
   Purge gas: nitrogen nitrogen purging speed: 50 mL/min
   Heating rate: 10.0 °C/min
   Temperature range: 30-350 °C
4. DVS, i.e., dynamic vapor sorption
   Surface Measurement Systems advantage 2 is used for detection at 25 °C. The humidity is 50%-95%-0%-95%-50% RH. The step is 10%. The criterion is that the mass change dM/dT for each step is less than 0.002%. TMAX is 360 min. Two cycles are carried out.
5. The progress of the reaction in the examples is monitored by means of thin layer chromatography (TLC). The developing solvent used in the reaction, the eluent system of column chromatography used in the purification of compounds and the developing solvent system of thin layer chromatography include: A: Dichloromethane/methanol system, B: n-hexane/ethyl acetate system. The silica gel plate for thin layer chromatography is Yantai Huanghai HSGF254 or Qingdao GF254 silica gel plate, the specification of the silica gel plate used for thin layer chromatography (TLC) is 0.15 mm to 0.2 mm, and the specification of the silica gel plate used for separating and purifying products by thin layer chromatography is 0.4 mm to 0.5 mm. For silica gel column chromatography, Yantai Huanghai silica gel 200-300 mesh silica gel is generally used as a carrier.
6. The structures of the compounds were determined by nuclear magnetic resonance (NMR) spectroscopy and/or mass spectrometry (MS). NMR shifts (δ) are given in a unit of 10-6 (ppm).
   NMR spectra were measured using a Bruker AVANCE-400 nuclear magnetic resonance instrument, with deuterated dimethyl sulfoxide (DMSO-d6), deuterated chloroform (CDCl3) and deuterated methanol (CD3OD) as solvents and tetramethylsilane (TMS) as an internal standard.
   MS determination was carried out by means of Agilent 1200/1290 DAD-6110/6120 Quadrupole MS liquid chromatography-mass spectrometer (manufacturer: Agilent, MS model: 6110/6120 Quadrupole MS); waters ACQuity UPLC-QD/SQD (manufacturer: waters, MS model: waters ACQuity Qda Detector/waters SQ Detector); and THERMO Ultimate 3000-Q Exactive (manufacturer: THERMO, MS model: THERMO Q Exactive).
7. Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.
8. HPLC determination was carried out by means of Agilent 1260DAD highperformance liquid chromatograph (ACE Excel C18 150 × 4.6 mm chromatographic column) and Thermo Dionex Ultimate 3000 high-pressure liquid chromatograph (Waters Xbridge C18 150 × 4.6 mm chromatographic column).

### Example 1

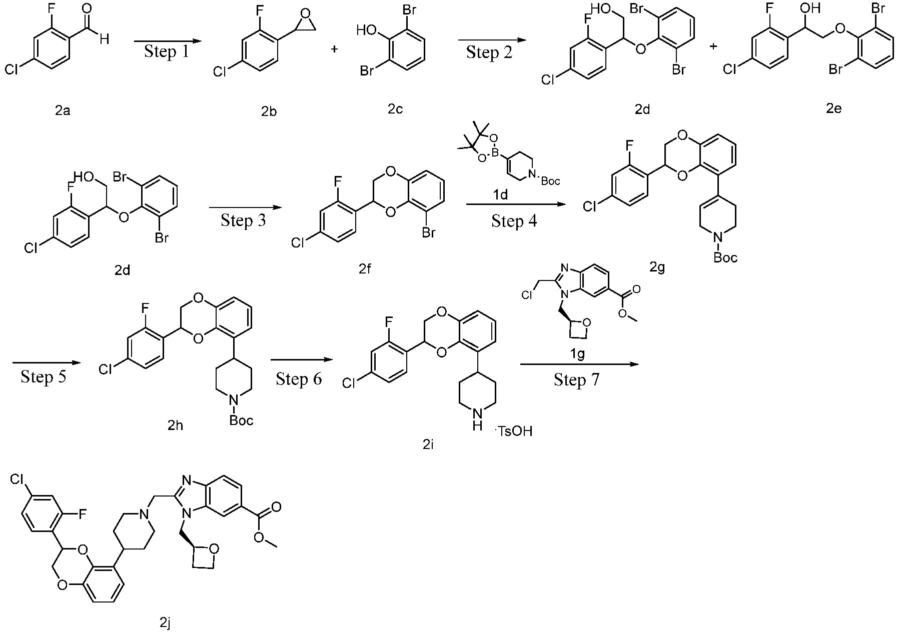

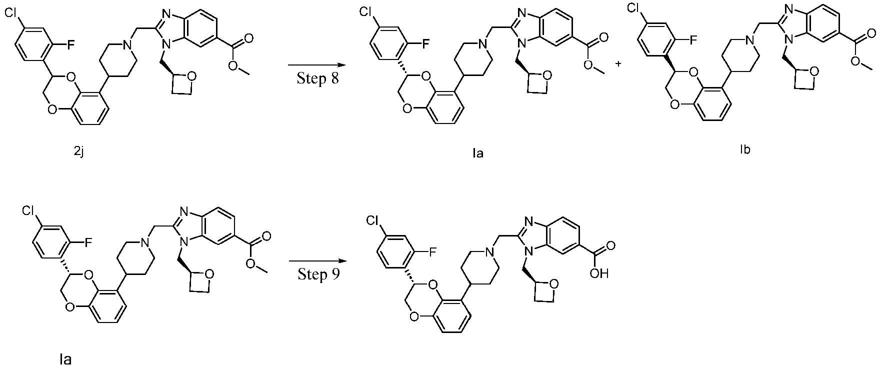

### Step 1

### 2-(4-Chloro-2-fluorophenyl)oxirane 2b

Potassium tert-butoxide (1.70 g, 15.14 mmol, Accela ChemBio (Shanghai) Inc.) was added to tetrahydrofuran (30 mL). Trimethylsulfonium iodide (3.09 g, 15.14 mmol, Adamas Reagent Co., Ltd.) was added under ice bath conditions. The mixture was stirred for 5 min. 4-Chloro-2-fluorobenzaldehyde **2a** (2.0 g, 12.61 mmol, Accela ChemBio (Shanghai) Inc.) was added. The mixture was filtered, diluted with ethyl acetate (80 mL), washed with saturated aqueous ammonium chloride solution (30 mL×2), washed with saturated brine (30 mL×2), dried over anhydrous sodium sulfate and concentrated under reduced pressure to remove the solvent. The resulting residue was purified by silica gel column chromatography with eluent system B to give the title compound **2b** (650 mg, yield:29.9%).

¹ H NMR (500 MHz, CDCl₃) δ 7.05-7.13 (m, 3H), 4.01-4.15 (m, 1H), 3.17 (dd, 1H), 3.75 (dd, 1H).

### Step 2

### 2-(4-Chloro-2-fluorophenyl)-2-(2,6-dibromophenoxy)ethanol 2d

### 1-(4-Chloro-2-fluorophenyl)-2-(2,6-dibromophenoxy)ethanol 2e

After compound **2b** (520 mg, 3.01 mmol) and 2,6-dibromophenol **2c** (759 mg, 3.01 mmol, TCI (Shanghai) Co., Ltd.) were mixed, sodium methoxide (16 mg, 0.30 mmol, Adamas Reagent Co., Ltd.) was added.The mixture was stirred at 130 °C. for 2 h. After cooling, the resulting residue was purified by silica gel column chromatography with eluent system B to give the title compound **2d** (210 mg, yield: 16.4%) and compound **2e** (140 mg, yield: 10.9%).

**2d** MS m/z (ESI): 422.9 [M-1].

**2d** ¹ H NMR (500 MHz, DMSO-d₆) δ 7.69 (t, 1H), 7.63 (d, 2H), 7.40 (dd, 1H), 7.35 (dd, 1H), 7.00 (t, 1H), 5.59 (t, 1H), 5.02 (t, 1H), 3.98-4.03 (m, 1H), 3.81-3.90 (m, 1H).

**2e** ¹ H NMR (500 MHz, DMSO-d₆) δ 7.64 (d, 2H), 7.62 (t, 1H), 7.39 (dd, 1H), 7.32 (dd, 1H), 7.02 (t, 1H), 5.82-6.01 (m, 1H), 5.28 (t, 1H), 4.07-4.12 (m, 1H), 3.95-4.00 (m, 1H).

### Step 3

### 8-Bromo-2-(4-chloro-2-fluorophenyl)-2,3-dihydrobenzo[b][1,4]dioxane 2f

Compound **2d** (595 mg, 1.40 mmol) was dissolved in anhydrous toluene (8 mL). S-1,1'-Bi-2-naphthol (159 mg, 0.55 mmol, Accela ChemBio (Shanghai) Inc.), cuprous iodide (52 mg, 0.27 mmol, Sinopharm Chemical Reagent Co., Ltd.) and cesium carbonate (912 mg, 2.80 mmol, Accela ChemBio (Shanghai) Inc.) were successively added. The mixture was heated at reflux and stirred for 18 h, cooled and concentrated under reduced pressure to remove the solvent. The resulting residue was purified by silica gel column chromatography with eluent system B to give the title compound **2f** (380 mg, yield: 78.9%).

MS m/z (ESI): 343.1 [M-1].

¹ HNMR (500 MHz, DMSO-d₆) δ 7.57 (dd, 1H), 7.54 (t, 1H), 7.43 (dd, 1H), 7.19 (dd, 1H), 6.98 (dd, 1H), 6.85 (t, 1H), 5.58 (dd, 1H), 4.51 (dd, 1H), 4.20 (dd, 1H).

### Step 4

### tert-Butyl 4-(3-(4-chloro-2-fluorophenyl)-2,3-dihydrobenzo[b][1,4]dioxan-5-yl)-5,6-dihydropyridine-1 (2H)-carboxylate 2g

Compound **2f** (354 mg, 1.03 mmol) and compound **1d** (350 mg, 1.13 mmol, Accela ChemBio (Shanghai) Inc.) were dissolved in 24 mL of a mixed solution of 1,4-dioxane and water (V/V=5 : 1). Sodium carbonate (218 mg, 2.06 mmol), tetrakis(triphenylphosphine)palladium (119 mg, 1.03 mmol) were added. The mixture was stirred at 90 °C. for 4 h under nitrogen, cooled to room temperature, filtered and concentrated under reduced pressure to remove the solvent. The resulting residue was purified by silica gel column chromatography with eluent system B to give the title compound **2g** (410 mg, yield: 89.2%).

MS m/z (ESI): 390.1 [M-55].

¹ H NMR (500 MHz, CDCl₃) δ 7.39 (t, 1H), 7.19-7.23 (m, 1H), 7.15 (dd, 1H), 6.83-6.89 (m, 2H), 6.77-6.81 (m, 1H), 5.76-5.91 (m, 1H), 5.32-5.46 (m, 1H), 5.41 (dd, 1H), 3.99-4.08 (m, 2H), 3.97 (dd, 1H), 3.43-3.69 (m, 2H), 2.40-2.63 (m, 2H), 1.47 (s, 9H).

### Step 5

### tert-Butyl 4-(3-(4-chloro-2-fluorophenyl)-2,3-dihydrobenzo[b][1,4]dioxan-5-yl)piperidine-1-carboxylate 2h

Compound **2g** (220 mg, 0.49 mmol) was dissolved in ethyl acetate (10 mL) and 1,2-dichlorobenzene (0.5 mL, TCI (Shanghai) Co., Ltd.). 10% palladium on carbon (50 mg, 0.47 mmol) was added. Hydrogenation was performed at room temperature for 1 h under one atmosphere of hydrogen. The mixture was filtered and concentrated under reduced pressure to remove the solvent. The resulting residue was purified by silica gel column chromatography with eluent system B to give the title compound **2h** (178 mg, yield:80.5%).

MS m/z (ESI): 392.1[M-55].

¹ H NMR (500 MHz, CDCl₃) δ 7.40 (t, 1H), 7.21-7.24 (m, 1H), 7.16 (dd, 1H), 6.82-6.88 (m, 1H), 6.76-6.81 (m, 2H), 5.35-5.45 (m, 1H), 4.40 (dd, 1H), 4.09-4.33 (m, 2H), 3.96 (dd, 1H), 2.99-3.11 (m, 1H), 2.67-2.90 (m, 2H), 1.72-1.91 (m, 2H), 1.58-1.69 (m, 2H), 1.46 (s, 9H).

### Step 6

### 4-(3-(4-Chloro-2-fluorophenyl)-2,3-dihydrobenzo[b][1,4]dioxan-5-yl)piperidine 4-methylbenzenesulfonate 2i

Compound **2h** (178 mg, 0.40 mmol) was dissolved in ethyl acetate (5 mL). p-Toluenesulfonic acid monohydrate (189 mg, 0.99 mmol) was added. The mixture was stirred at 60 °C. for 2 h. The mixture was cooled to room temperature and concentrated under reduced pressure to give the crude title product **2i** (206 mg). The product was directly used in the next step without being purified.

MS m/z (ESI): 348.1 [M+1].

### Step 7

### Methyl 2-((4-(3-(4-chloro-2-fluorophenyl)-2,3-dihydrobenzo[b][1,4]dioxan-5-yl)piperidin-1-yl)methyl)-1-((S)-oxetan-2-ylmethyl)-1H-benzo[d]imidazole-6-carboxylate 2j

Compound **1g** (175 mg, 0.59 mmol) and **2i** (206 mg, 0.59 mmol) were dissolved in acetonitrile (10 mL). Potassium carbonate (410 mg, 2.97 mmol) was added. The mixture was stirred at 60 °C. for 3 h, filtered and concentrated under reduced pressure to remove the solvent. The resulting residue was purified by silica gel column chromatography with eluent system B to give the title mixture of diastereomers **2j** (207 mg, yield: 57.7%).

MS m/z (ESI): 606.2 [M+1].

### Step 8

### Methyl 2-((4-((R)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylate Ia

### Methyl 2-((4-((R)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylate Ib

Compound **2j** (830 mg, 1.37 mmol) was resolved by preparative chiral chromatography (resolution conditions: CHIRALPAK IG 250×20 mm, 5 µm (with column protection); mobile phase: hexane/EtOH (0.1% DEA)=70/30 (V/V), flow rate: 20 mL/min). The corresponding fractions were collected and concentrated under reduced pressure to give the title products (415 mg and 340 mg).

Single-configuration compound (shorter retention time **Ia**): MS m/z (ESI): 606.0 [M+1]. Preparative chiral chromatography: retention time 13.653 min.

Single-configuration compound (longer retention time **Ib**): MS m/z (ESI): 606.0 [M+1]. Preparative chiral chromatography: retention time 16.422 min.

### Step 9

### 2-((4-((S)-3-(4-Chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid

**Ia** (415 mg, 0.68 mmol) was dissolved in 36 mL of a mixed solvent of acetonitrile and water (V : V = 6 : 1). Lithium hydroxide monohydrate (145 mg, 3.46 mmol) was added. The mixture was stirred at 40 °C. for 18 h. The reaction mixture was cooled to room temperature, adjusted to pH 5-6 with aqueous citric acid solution (1 M) and extracted with ethyl acetate (30 mL×3). The organic phase was concentrated under reduced pressure and then purified by high performance liquid chromatography (Gilson281, column: Boston Phlex C18 150×30 mm, 5 µm; mobile phase 1: water (containing 10 mmol/L ammonium bicarbonate); mobile phase 2: acetonitrile; 15 min of gradient elution: 30% to 50%, flow rate: 30 mL/min) to give the title product 4 (310 mg, yield: 76.46%).

MS m/z (ESI): 592.2 [M+1].

¹ H NMR (500 MHz, DMSO-d₆) δ 12.42-12.97 (brs, 1H), 8.20-8.28 (m, 1H), 7.74-7.83 (m, 1H), 7.61 (d, 1H), 7.55-7.58 (m, 1H), 7.48-7.54 (m, 1H), 7.38-7.44 (m, 1H), 6.70-6.90 (m, 3H), 5.40-7.49 (m, 1H), 5.01-5.13 (m, 1H), 4.72-4.84 (m, 1H), 4.59-4.67 (m, 1H), 4.39-4.51 (m, 2H), 4.31-4.38 (m, 1H), 4.04-4.13 (m, 1H), 3.86-3.95 (m, 1H), 3.71-3.79 (m, 1H), 2.91-3.01 (m, 1H), 2.77-2.88 (m, 2H), 2.61-2.72 (m, 1H), 2.33-2.44 (m, 1H), 2.07-2.25 (m, 2H), 1.73-1.81 (m, 1H), 1.63-1.73 (m, 2H), 1.54-1.63 (m, 1H).

Test Example 1: Evaluation of Agonist Activity Against GLP-1 Receptor

### I. Purpose

This experiment was intended to test the agonist activity of the compound molecules against the GLP-1 receptor and evaluate the in vitro activity of the molecules according to EC₅₀. The experiment adopted a ONE-Glo^{™} Luciferase Assay System (Promega, E6110). Under the action of compound molecules, GLP-1R downstream signaling pathways were activated to cause elevated cAMP level. The combination of cAMP and CRE could start the transcription expression of CRE downstream luciferase genes, the luciferase could emit fluorescence when reacting with substrates thereof, and the activity of the compound for agonizing GLP-1 receptors was reflected by measuring fluorescence signals through a ONE-Glo^{™} reagent.

### II. Method

Stably-expressed CHO-K1/CRE-luc/GLP-1 receptor cell strains (self-construction of GLP-1 receptor plasmid; CRE-luc plasmid Promega E8471) were constructed. CHO-K1/CRE-luc/GLP-1 receptor cells were digested, and resuspended after centrifugation. Single cell suspension was uniformly mixed, and adjusted to a viable cell density of 2.5 × 10⁵ cells/mL with a cell culture medium (DME/F-12+10% FBS), and the resulting solution was added to a 96-well cell culture plate at 90 µL/well (Corning, #3903). The plate was incubated in an incubator for 16 h (37 °C., 5% CO₂).

The compound was dissolved in DMSO to prepare a stock solution with an initial concentration of 20 mM. The starting concentration of the small molecule compound was 0.2 mM, and the compound underwent 3-fold serial dilution for a total of 10 concentration points, with DMSO at the 11th point. To another 96-well plate was added 95 µL of cell culture medium (DME/F-12+10% FBS), 5 µL of test samples with different concentrations were added to each well, followed by uniform mixing, and then 10 µL of test samples with different concentrations were added to each well, with two duplicate wells set for each sample. The plate was incubated in an incubator for 6 h (37 °C., 5% CO₂). The 96-well cell culture plate was taken out, and 100 µL of ONE-Glo^{™} reagent was added to each well, followed by incubation at room temperature for 10 min. The plate was placed in a microplate reader (EnVision 2105, PE) for determination of chemiluminescence.

### III. Data Analysis

Data were processed and analyzed using Microsoft Excel and Graphpad Prism 5. EC50 values of the compounds were obtained.

**Table 1**

| Compound | EC₅₀ (nM) | Emax% |
|---|---|---|
| A | 0.74 | 103.02 |

### Example 2: Preparation of amorphous form of Compound A

10 mg of Compound A was weighed, 500 µL of water was added, and the mixture was pulped at room temperature, centrifuged and dried to obtain a solid. After detection by X-ray powder diffraction, the X-ray powder diffraction pattern expressed with diffraction angle 2*θ* had no obvious characteristic peaks, and the XRPD pattern was as shown in FIG. 1. The product was in an amorphous form.

### Example 3: Preparation of crystal form A of Compound A

10 mg of Compound A was weighed, 500 µL of n-propanol was added, the sample was dissolved at 60 °C until clear and cooled for precipitation, and after centrifugation and drying, a solid was obtained. After detection by X-ray powder diffraction, the XRPD pattern was as shown in FIG. 2, and the positions of the characteristic peaks thereof were as shown in Table 2. The product was designated as crystal form A. The DSC pattern showed that the peak value of an endothermic peak was 197.60 °C. The TGA pattern showed that the weight loss at 30-150 °C was 0.35%.

**Table 2**

| **Peak No.** | **20 value [° or degrees]** | **d[Å]** | **Relative intensity %** |
|---|---|---|---|
| 1 | 7.654 | 11.54175 | 24.6 |
| 2 | 9.587 | 9.21773 | 76.5 |
| 3 | 10.216 | 8.65141 | 64.3 |
| 4 | 11.812 | 7.48602 | 85.1 |
| 5 | 12.645 | 6.99499 | 53.0 |
| 6 | 13.956 | 6.34043 | 59.5 |
| 7 | 15.488 | 5.71666 | 32.9 |
| 8 | 16.503 | 5.36732 | 14.0 |
| 9 | 17.541 | 5.05178 | 34.1 |
| 10 | 18.204 | 4.86926 | 100.0 |
| 11 | 19.462 | 4.55749 | 48.1 |
| 12 | 20.041 | 4.42700 | 31.4 |
| 13 | 20.697 | 4.28805 | 20.9 |
| 14 | 21.477 | 4.13410 | 25.3 |
| 15 | 21.812 | 4.07140 | 12.3 |
| 16 | 22.615 | 3.92861 | 5.6 |
| 17 | 23.404 | 3.79792 | 70.4 |
| 18 | 24.533 | 3.62571 | 12.0 |
| 19 | 26.618 | 3.34613 | 18.0 |
| 20 | 28.168 | 3.16549 | 14.2 |
| 21 | 29.406 | 3.03493 | 20.6 |
| 22 | 31.044 | 2.87844 | 6.1 |

### Example 4: Preparation of crystal form A of Compound A

10 mg of Compound A was weighed, and 100 µL of THF was added. After circulating heating and cooling between 50 °C and 5 °C, no precipitation occurred, and the sample was volatilized at room temperature to obtain a solid. After detection by X-ray powder diffraction, the product was crystal form A.

### Example 5: Preparation of crystal form A of Compound A

10 mg of Compound A was weighed, and 500 µL of nitromethane was added. After circulating heating and cooling between 50 °C and 5 °C, a solid was precipitated, centrifuged and dried to obtain a solid. After detection by X-ray powder diffraction, the product was crystal form A.

### Example 6: Preparation of crystal form A of Compound A

2 g of Compound A was weighed, and 50 mL of n-propanol was added and stirred at 65 °C until dissolved clear. After heating was stopped, the product was slowly cooled to 35 °C or less, 5 mg of seed crystal was added and stirred, and after stirring, a solid was precipitated. After continued stirring, it was transferred to 2-8 °C and stirred overnight, and after centrifugation and drying, a solid was obtained. After detection by X-ray powder diffraction, the product was crystal form A.

### Example 7: Preparation of crystal form A of Compound A

30 mg of Compound A was weighed and dispersed in 0.3 mL of isopropanol. The solution was white and turbid. It was pulped at room temperature for 3 days and filtered, and the filter cake was collected and dried in vacuum. The product was in crystal form A.

### Example 8: Preparation of crystal form A of Compound A

17 g of crude Compound A was weighed, added to 180 mL of ethyl acetate, heated to 80 °C, and stirred. A solid was gradually precipitated during the stirring process. 90 mL of n-hexane was slowly dropwise added. After addition, it was stirred at 80 °C for 30 minutes, naturally cooled to room temperature, then stirred in an ice bath for 30 minutes, and filtered, and the filter cake was collected and dried in vacuum. The product was crystal form A.

### Example 9: Preparation of crystal form A of Compound A

30 mg of Compound A was weighed and dispersed in 0.3 mL of isopropyl acetate. The solution was white and turbid. It was pulped at room temperature for 3 days and filtered, and the filter cake was collected and dried in vacuum. The product was in crystal form A.

### Example 10: Preparation of crystal form B1 of Compound A

10 mg of Compound A was weighed, and 500 µL of acetone was added. The sample was dissolved at 50 °C until clear. Circulating heating and cooling between 50 °C and 5 °C was performed for volatilization crystallization. After detection by X-ray powder diffraction, the XRPD pattern was as shown in FIG. 3, and the positions of the characteristic peaks thereof were as shown in Table 3. The product was designated as crystal form B1. The DSC pattern showed that the peak values of endothermic peaks were 129.48 °C and 140.82 °C. The TGA pattern showed that the weight loss at 30-160 °C was 4.62%.

**Table 3**

| **Peak No.** | **20 value [° or degrees]** | **d[Å]** | **Relative intensity %** |
|---|---|---|---|
| 1 | 8.135 | 10.85958 | 46.1 |
| 2 | 8.915 | 9.91088 | 40.9 |
| 3 | 10.507 | 8.41313 | 36.5 |
| 4 | 11.259 | 7.85263 | 77.1 |
| 5 | 11.508 | 7.68352 | 64.5 |
| 6 | 12.223 | 7.23554 | 32.1 |
| 7 | 13.632 | 6.49039 | 23.5 |
| 8 | 15.055 | 5.88013 | 2.5 |
| 9 | 16.751 | 5.28822 | 30.1 |
| 10 | 17.836 | 4.96907 | 29.1 |
| 11 | 19.024 | 4.66137 | 100.0 |
| 12 | 19.914 | 4.45498 | 18.2 |
| 13 | 20.541 | 4.32025 | 27.5 |
| 14 | 22.205 | 4.00029 | 23.2 |
| 15 | 22.736 | 3.90802 | 31.4 |
| 16 | 23.519 | 3.77967 | 11.9 |
| 17 | 24.110 | 3.68831 | 15.3 |
| 18 | 24.608 | 3.61480 | 13.2 |
| 19 | 25.271 | 3.52141 | 46.1 |
| 20 | 26.849 | 3.31790 | 26.9 |
| 21 | 31.479 | 2.83969 | 12.7 |
| 22 | 33.974 | 2.63663 | 7.4 |

### Example 11: Preparation of crystal form B1 of Compound A

10 mg of Compound A was weighed, and 500 µL of isopropanol was added. After cooling, a solid was precipitated, and after centrifugation and drying, a solid was obtained. After detection by X-ray powder diffraction, the product was crystal form B1.

### Example 12: Preparation of crystal form B1 of Compound A

10 mg of Compound A was weighed, and 500 µL of isopentanol was added. After cooling, a solid was precipitated, and after centrifugation and drying, a solid was obtained. After detection by X-ray powder diffraction, the product was crystal form B1.

### Example 13: Preparation of crystal form B1 of Compound A

10 mg of Compound A was weighed, and 150 µL of THF was added. After it was dissolved clear, water was added to make up to 600 µL, whereupon a solid was precipitated, and after centrifugation and drying, a solid was obtained. After detection by X-ray powder diffraction, the product was crystal form B1.

### Example 14: Preparation of crystal form B1 of Compound A

10 mg of Compound A was weighed, and 100 µL of 1,2-dichloroethane was added. After cooling, a solid was precipitated, and after centrifugation and drying, a solid was obtained. After detection by X-ray powder diffraction, the product was crystal form B1.

### Example 15: Preparation of crystal form B2 of Compound A

10 mg of Compound A was weighed, and 100 µL of methyl isobutyl ketone was added. After cooling, a solid was precipitated, and after centrifugation and drying, a solid was obtained. After detection by X-ray powder diffraction, the XRPD pattern was as shown in FIG. 4, and the positions of the characteristic peaks thereof were as shown in Table 4. The product was designated as crystal form B2. The DSC pattern showed that the peak value of an endothermic peak was 137.59 °C. The TGA pattern showed that the weight loss at 30-160 °C was 9.27%.

**Table 4**

| **Peak No.** | **20 value [° or degrees]** | **d[Å]** | **Relative intensity %** |
|---|---|---|---|
| 1 | 8.182 | 10.79687 | 26.8 |
| 2 | 8.839 | 9.99636 | 23.1 |
| 3 | 10.401 | 8.49870 | 27.6 |
| 4 | 11.168 | 7.91604 | 38.0 |
| 5 | 11.679 | 7.57135 | 19.1 |
| 6 | 13.714 | 6.45179 | 21.9 |
| 7 | 14.880 | 5.94899 | 11.8 |
| 8 | 16.592 | 5.33854 | 16.1 |
| 9 | 17.660 | 5.01800 | 5.4 |
| 10 | 18.906 | 4.69018 | 100.0 |
| 11 | 20.245 | 4.38291 | 28.4 |
| 12 | 21.895 | 4.05608 | 22.0 |
| 13 | 22.600 | 3.93119 | 18.2 |
| 14 | 23.175 | 3.83491 | 2.6 |
| 15 | 23.950 | 3.71262 | 8.6 |
| 16 | 25.134 | 3.54026 | 23.6 |
| 17 | 26.025 | 3.42103 | 0.7 |
| 18 | 28.783 | 3.09926 | 1.0 |

### Example 16: Preparation of crystal form B3 of Compound A

10 mg of Compound A was weighed, and 500 µL of ethyl acetate was added, whereupon it was dissolved clear. After stirring, precipitation occurred, and after centrifugation and drying, a solid was obtained. After detection by X-ray powder diffraction, the XRPD pattern was as shown in FIG. 5, and the positions of the characteristic peaks thereof were as shown in Table 5. The product was designated as crystal form B3. The DSC pattern showed that the peak values of endothermic peaks were 114.84 °C and 146.66 °C. The TGA pattern showed that the weight loss at 30-160 °C was 2.08%.

**Table 5**

| **Peak No.** | **20 value [° or degrees]** | **d[Å]** | **Relative intensity %** |
|---|---|---|---|
| 1 | 8.224 | 10.74250 | 31.6 |
| 2 | 8.976 | 9.84452 | 36.5 |
| 3 | 10.548 | 8.38013 | 87.2 |
| 4 | 11.269 | 7.84568 | 86.8 |
| 5 | 11.496 | 7.69111 | 100.0 |
| 6 | 12.264 | 7.21142 | 27.2 |
| 7 | 13.730 | 6.44439 | 20.7 |
| 8 | 14.829 | 5.96916 | 26.0 |
| 9 | 16.669 | 5.31414 | 10.2 |
| 10 | 17.557 | 5.04732 | 61.0 |
| 11 | 18.103 | 4.89643 | 42.7 |
| 12 | 19.135 | 4.63460 | 87.9 |
| 13 | 19.751 | 4.49135 | 72.0 |
| 14 | 20.605 | 4.30700 | 71.5 |
| 15 | 21.347 | 4.15897 | 11.9 |
| 16 | 22.246 | 3.99299 | 13.2 |
| 17 | 22.767 | 3.90268 | 47.3 |
| 18 | 23.522 | 3.77909 | 29.9 |
| 19 | 24.738 | 3.59607 | 35.0 |
| 20 | 25.360 | 3.50931 | 50.4 |
| 21 | 26.556 | 3.35389 | 36.2 |
| 22 | 26.893 | 3.31261 | 26.8 |
| 23 | 28.101 | 3.17286 | 14.7 |

### Example 17: Preparation of crystal form B3 of Compound A

10 mg of Compound A was weighed, and 100 µL of dichloromethane was added, whereupon it was dissolved clear. After cooling, a solid was precipitated, and after centrifugation and drying, a solid was obtained. After detection by X-ray powder diffraction, the product was crystal form B3.

### Example 18: Preparation of crystal form B3 of Compound A

10 mg of Compound A was weighed, and 100 µL of 10% water/acetone solution was added, whereupon it was dissolved clear. After cooling, a solid was precipitated, and after centrifugation and drying, a solid was obtained. After detection by X-ray powder diffraction, the product was crystal form B3.

### Example 19: Preparation of crystal form B1 of Compound A

10 mg of Compound A was weighed, and 500 µL of isopropyl acetate was added, whereupon it was dissolved clear. After stirring, precipitation occurred, and after centrifugation and drying, a solid was obtained. After detection by X-ray powder diffraction, the product was crystal form B1.

### Example 20: Preparation of crystal form B1 of Compound A

10 mg of Compound A was weighed, and 100 µL of propylene glycol methyl ether was added. After circulating heating and cooling between 50 °C and 5 °C, a solid was precipitated, centrifuged and dried to obtain a solid. After detection by X-ray powder diffraction, the product was crystal form B1.

### Example 21: Preparation of crystal form B1 of Compound A

10 mg of Compound A was weighed, and 100 µL of p-xylene was added. After circulating heating and cooling between 50 °C and 5 °C, a solid was precipitated, centrifuged and dried to obtain a solid. After detection by X-ray powder diffraction, the product was crystal form B1.

### Example 22: Preparation of crystal form C of Compound A

10 mg of Compound A was weighed, 500 µL of toluene was added, and the mixture was pulped at room temperature, centrifuged and dried to obtain a solid. After detection by X-ray powder diffraction, the product was crystal form C, the XRPD pattern was as shown in FIG. 6, and the positions of the characteristic peaks thereof were as shown in Table 6.

**Table 6**

| **Peak No.** | **20 value [° or degrees]** | **d[Å]** | **Relative intensity %** |
|---|---|---|---|
| 1 | 5.470 | 16.14310 | 20.3 |
| 2 | 10.094 | 8.75591 | 49.5 |
| 3 | 11.511 | 7.68097 | 100.0 |
| 4 | 12.138 | 7.28566 | 27.3 |
| 5 | 14.975 | 5.91126 | 21.5 |
| 6 | 15.875 | 5.57795 | 37.7 |
| 7 | 17.378 | 5.09893 | 47.4 |
| 8 | 17.763 | 4.98926 | 30.1 |
| 9 | 18.573 | 4.77354 | 34.6 |
| 10 | 19.413 | 4.56869 | 27.7 |
| 11 | 20.113 | 4.41124 | 63.2 |
| 12 | 22.925 | 3.87615 | 31.7 |
| 13 | 23.881 | 3.72306 | 26.7 |
| 14 | 24.293 | 3.63152 | 10.2 |
| 15 | 26.177 | 3.40158 | 17.5 |
| 16 | 28.163 | 3.16606 | 17.7 |
| 17 | 28.717 | 3.10617 | 10.5 |
| 18 | 32.762 | 2.73133 | 3.2 |

### Example 23: Preparation of crystal form D of Compound A

10 mg of Compound A was weighed, 500 µL of methanol was added, and the mixture was pulped at room temperature, centrifuged and dried to obtain a solid. After detection by X-ray powder diffraction, the XRPD pattern was as shown in FIG. 7, and the positions of the characteristic peaks thereof were as shown in Table 7. The product was designated as crystal form D. The DSC pattern showed that the peak value of an endothermic peak was 190.72 °C. The TGA pattern showed that the weight loss at 30-220 °C was 1.88%.

**Table 7**

| **Peak No.** | **20 value [° or degrees]** | **d[Å]** | **Relative intensity %** |
|---|---|---|---|
| 1 | 6.343 | 13.92358 | 26.2 |
| 2 | 10.940 | 8.08076 | 100.0 |
| 3 | 12.216 | 7.23929 | 81.2 |
| 4 | 12.762 | 6.93106 | 11.3 |
| 5 | 14.108 | 6.27234 | 6.0 |
| 6 | 14.684 | 6.02784 | 13.4 |
| 7 | 16.167 | 5.47814 | 16.7 |
| 8 | 16.510 | 5.36508 | 10.3 |
| 9 | 17.695 | 5.00836 | 22.9 |
| 10 | 18.344 | 4.83242 | 98.7 |
| 11 | 18.973 | 4.67364 | 28.7 |
| 12 | 19.472 | 4.55500 | 25.7 |
| 13 | 19.931 | 4.45122 | 40.8 |
| 14 | 21.753 | 4.08237 | 34.6 |
| 15 | 22.979 | 3.86716 | 39.2 |
| 16 | 23.464 | 3.78836 | 9.8 |
| 17 | 24.025 | 3.70110 | 6.7 |
| 18 | 24.306 | 3.65900 | 11.2 |
| 19 | 24.685 | 3.60363 | 30.0 |
| 20 | 25.898 | 3.43751 | 16.3 |
| 21 | 26.582 | 3.35059 | 3.4 |
| 22 | 27.237 | 3.27149 | 8.5 |
| 23 | 29.077 | 3.06854 | 0.7 |
| 24 | 30.824 | 2.89855 | 3.3 |
| 25 | 33.069 | 2.70669 | 5.0 |

### Example 24: Preparation of crystal form D of Compound A

10 mg of Compound A was weighed, and 500 µL of a mixed solution of methanol : water = 1 : 1 was added. After circulating heating and cooling between 50 °C and 5 °C, a solid was precipitated, centrifuged and dried to obtain a solid. After detection by X-ray powder diffraction, the product was crystal form D.

### Example 25: Preparation of crystal form D of Compound A

10 mg of Compound A was weighed, and 150 µL of THF was added for dissolution. Methanol was added to make up to 800 µL, whereupon, a solid was precipitated, centrifuged and dried to obtain a solid. After detection by X-ray powder diffraction, the product was crystal form D.

### Example 26: Preparation of crystal form D of Compound A

10 mg of Compound A was weighed, and 500 µL of a mixed solution of acetonitrile : methanol = 1 : 1 was added. After circulating heating and cooling between 50 °C and 5 °C, a solid was precipitated, centrifuged and dried to obtain a solid. After detection by X-ray powder diffraction, the product was crystal form D.

### Example 27: Preparation of crystal form D of Compound A

10 mg of Compound A was weighed, and 50 µL of chloroform was added for dissolution. Methanol was added to make up to 800 µL, whereupon, a solid was precipitated, centrifuged and dried to obtain a solid. After detection by X-ray powder diffraction, the product was crystal form D.

### Example 28: Preparation of crystal form E of Compound A

10 mg of Compound A was weighed, and 500 µL of an acetonitrile mixed solution was added. After circulating heating and cooling between 50 °C and 5 °C, a solid was precipitated, centrifuged and dried to obtain a solid. After detection by X-ray powder diffraction, the XRPD pattern was as shown in FIG. 8, and the positions of the characteristic peaks thereof were as shown in Table 8. The DSC pattern showed that the peak values of endothermic peaks were 118.74 °C and 125.07 °C. The product was designated as crystal form E. The TGA pattern showed that the weight loss at 30-170 °C was 3.50%.

**Table 8**

| **Peak No.** | **20 value [° or degrees]** | **d[Å]** | **Relative intensity %** |
|---|---|---|---|
| 1 | 8.245 | 10.71477 | 11.5 |
| 2 | 8.738 | 10.11147 | 9.0 |
| 3 | 9.261 | 9.54200 | 15.3 |
| 4 | 10.735 | 8.23484 | 24.4 |
| 5 | 11.591 | 7.62824 | 32.8 |
| 6 | 12.056 | 7.33507 | 8.6 |
| 7 | 13.946 | 6.34511 | 12.0 |
| 8 | 14.925 | 5.93114 | 8.6 |
| 9 | 15.615 | 5.67044 | 1.8 |
| 10 | 16.486 | 5.37267 | 5.6 |
| 11 | 16.922 | 5.23533 | 9.3 |
| 12 | 17.645 | 5.02222 | 36.7 |
| 13 | 18.291 | 4.84642 | 16.8 |
| 14 | 19.060 | 4.65253 | 54.3 |
| 15 | 20.066 | 4.42151 | 100.0 |
| 16 | 20.667 | 4.29429 | 32.2 |
| 17 | 21.340 | 4.16027 | 1.6 |
| 18 | 22.474 | 3.95301 | 12.2 |
| 19 | 23.550 | 3.77474 | 6.6 |
| 20 | 24.608 | 3.61480 | 9.2 |
| 21 | 25.620 | 3.47417 | 7.9 |
| 22 | 26.987 | 3.30126 | 37.7 |
| 23 | 28.167 | 3.16563 | 7.4 |

### Example 29: Preparation of crystal form E of Compound A

10 mg of Compound A was weighed, and 150 µL of THF was added. After it was dissolved, acetonitrile was added to make up to 800 µL, whereupon a solid was precipitated, and after centrifugation and drying, a solid was obtained. After detection by X-ray powder diffraction, the product was crystal form E.

### Example 30: Preparation of crystal form F of Compound A

10 mg of Compound A was weighed, and 50 µL of DMSO was added. After it was dissolved, water was added to make up to 800 µL, whereupon a solid was precipitated, and after centrifugation and drying, a solid was obtained. After detection by X-ray powder diffraction, the product was crystal form F, the XRPD pattern was as shown in FIG. 9, and the positions of the characteristic peaks thereof were as shown in Table 9. The DSC pattern showed that the peak value of an endothermic peak was 111.89 °C. The TGA pattern showed that the weight loss at 30-150 °C was 3.16%.

**Table 9**

| **Peak No.** | **20 value [° or degrees]** | **d[Å]** | **Relative intensity %** |
|---|---|---|---|
| 1 | 9.543 | 9.26054 | 72.3 |
| 2 | 11.421 | 7.74124 | 41.9 |
| 3 | 14.557 | 6.08008 | 21.1 |
| 4 | 16.175 | 5.47532 | 23.6 |
| 5 | 17.886 | 4.95510 | 5.4 |
| 6 | 19.405 | 4.57054 | 100.0 |
| 7 | 22.153 | 4.00940 | 63.3 |
| 8 | 25.821 | 3.44759 | 36.3 |

### Example 31: Preparation of crystal form F of Compound A

10 mg of Compound A was weighed, and 50 µL of DMSO was added. After circulating heating and cooling between 50 °C and 5 °C, no precipitation occurred, and the sample was volatilized at room temperature to obtain a solid. After detection by X-ray powder diffraction, the product was crystal form F.

### Example 32: Preparation of crystal form G of Compound A

2 g of Compound A was weighed, 50 ml of n-propanol was added, the mixture was stirred at 65 °C until dissolved clear, slowly cooled to 5 °C, stirred overnight, and centrifuged, and the solid was dried at 60 °C in vacuum for 1 h. After detection by X-ray powder diffraction, the XRPD pattern was as shown in FIG. 10, and the positions of the characteristic peaks thereof were as shown in Table 10. The product was designated as crystal form G. The DSC pattern showed that the peak values of endothermic peaks were 115.06 °C and 200.24 °C. The TGA pattern showed that the weight loss at 30-150 °C was 7.35%.

**Table 10**

| **Peak No.** | **20 value [° or degrees]** | **d[Å]** | **Relative intensity %** |
|---|---|---|---|
| 1 | 6.120 | 14.43058 | 23.4 |
| 2 | 7.360 | 12.00113 | 4.7 |
| 3 | 9.096 | 9.71391 | 56.6 |
| 4 | 9.519 | 9.28328 | 21.6 |
| 5 | 11.107 | 7.95964 | 47.9 |
| 6 | 12.302 | 7.18895 | 28.9 |
| 7 | 13.387 | 6.60877 | 28.9 |
| 8 | 14.833 | 5.96761 | 21.8 |
| 9 | 17.239 | 5.13982 | 100.0 |
| 10 | 17.744 | 4.99446 | 79.9 |
| 11 | 18.958 | 4.67741 | 9.0 |
| 12 | 19.627 | 4.51939 | 3.3 |
| 13 | 20.302 | 4.37059 | 15.0 |
| 14 | 20.905 | 4.24600 | 11.2 |
| 15 | 22.416 | 3.96309 | 22.0 |
| 16 | 22.984 | 3.86631 | 27.0 |
| 17 | 23.342 | 3.80789 | 14.7 |
| 18 | 23.981 | 3.70780 | 31.5 |
| 19 | 24.814 | 3.58524 | 4.5 |
| 20 | 25.104 | 3.54450 | 5.5 |
| 21 | 25.879 | 3.44008 | 24.3 |
| 22 | 28.791 | 3.09833 | 12.2 |
| 23 | 29.663 | 3.00926 | 2.1 |
| 24 | 30.304 | 2.94708 | 8.5 |

### Test example 2: Study on hygroscopicity of crystal forms of Compound A

Surface Measurement Systems intrinsic DVS was used at 25 °C. The observed humidity ranged from 0% to 95%. The step was 10%. The criterion was that the mass change dM/dT for each step was less than 0.002%. TMAX was 360 min. Two cycles were carried out.

**Table 11**

| Test article | 0.0% RH to 80.0% RH | Crystal form |
|---|---|---|
| Crystal form A of Compound A | 0.65% | Unchanged |
| Crystal form D of Compound A | 1.95% | Unchanged |

### Test example 3: Study on stability of crystal forms of Compound A

A sample of a crystal form of Compound A was placed open and spread, and the stability of the sample was investigated under the conditions of illumination (4500 Lux), high temperature (40 °C and 60 °C) and high humidity (75% RH and 92.5% RH), respectively. The sampling and investigation period was 30 days.

**Table 12. Stability data of crystal form A**

| Condition | Time (days) | Purity % | Crystal form |
|---|---|---|---|
| Initial condition | 0 | 98.7 | A |
| Illumination (4500 Lux) | 5 | 98.7 | A |
| | 10 | 98.7 | A |
| | 30 | 98.8 | A |
| 40 °C | 5 | 98.8 | A |
| | 10 | 98.8 | A |
| | 30 | 98.8 | A |
| 60 °C | 5 | 98.8 | A |
| | 10 | 98.7 | A |
| | 30 | 98.8 | A |
| 75% RH | 5 | 98.7 | A |
| | 10 | 98.7 | A |
| | 30 | 98.7 | A |
| 92.5% RH | 5 | 98.8 | A |
| | 10 | 98.7 | A |
| | 30 | 98.8 | A |

**Table 13. Stability data of crystal form D**

| Condition | Time (days) | Purity % | Crystal form |
|---|---|---|---|
| Initial condition | 0 | 98.5 | D |
| Illumination (4500 Lux) | 5 | 98.5 | D |
| | 10 | 98.6 | D |
| | 30 | 98.6 | D |
| 40 °C | 5 | 98.4 | D |
| | 10 | 98.5 | D |
| | 30 | 98.5 | D |
| 60 °C | 5 | 98.5 | D |
| | 10 | 98.6 | D |
| | 30 | 98.6 | D |
| 75% RH | 5 | 98.5 | D |
| | 10 | 98.6 | D |
| | 30 | 98.5 | D |
| 92.5% RH | 5 | 98.6 | D |
| | 10 | 98.6 | D |
| | 30 | 98.5 | D |

The experimental results showed that crystal forms A and D of Compound A had good physical and chemical stability.

### Test example 4: Long-term/accelerated stability

A sample of a crystal form of Compound A was sealed with an aluminum foil bag and placed under the conditions of 25 °C/60% RH and 40 °C/75% RH, respectively, to investigate the stability. The results were as shown below.

**Table 14**

| | | | | | | |
|---|---|---|---|---|---|---|
| Sample | Placement condition | Purity % | | | | Crystal form |
| Crystal | | Initial | 1 month | 3 months | 6 months | / |
| form A | | condition | | | | |
| | 25 °C/60% RH | 99.5 | 99.5 | 99.5 | 99.5 | Unchanged |
| | 40 °C/75% RH | | 99.5 | 99.5 | 99.5 | Unchanged |
| Crystal form D | Placement condition | Purity % | | | | Crystal form |
| | | Initial condition | 1 month | 2 months | 3 months | / |
| | 25 °C/60% RH | 98.5 | 98.6 | 98.6 | 98.5 | Unchanged |
| | 40 °C/75% RH | | 98.6 | 98.4 | 98.3 | Unchanged |

The experimental results indicated that crystal form A of Compound A had good physical and chemical stability after being placed for 6 months under long-term accelerated conditions. Crystal form D had good physical and chemical stability after being placed for 3 months under long-term accelerated conditions.
The present invention further provides the following aspects:
1. An amorphous form of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has no obvious characteristic peaks, and preferably, the X-ray powder diffraction pattern expressed with diffraction angle 2*θ* is as shown in FIG. 1.
2. Crystal form A of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 9.587, 10.216, 11.812, 18.204, and 23.404; preferably characteristic peaks at 9.587, 10.216, 11.812, 12.645, 13.956, 15.488, 17.541, 18.204, 19.462, and 23.404; more preferably characteristic peaks at 7.654, 9.587, 10.216, 11.812, 12.645, 13.956, 15.488, 16.503, 17.541, 18.204, 19.462, 20.041, 20.697, 21.477, 21.812, 22.615, 23.404, 24.533, 26.618, 28.168, 29.406, and 31.044; and most preferably, the X-ray powder diffraction pattern expressed with diffraction angle 2*θ* is as shown in FIG. 2.
3. Crystal form B1 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 8.135, 8.915, 11.259, 11.508, 19.024, and 25.271; preferably characteristic peaks at 8.135, 8.915, 10.507, 11.259, 11.508, 12.223, 16.751, 19.024, 22.736, and 25.271; more preferably characteristic peaks at 8.135, 8.915, 10.507, 11.259, 11.508, 12.223, 13.632, 15.055, 16.751, 17.836, 19.024, 20.541, 22.205, 22.736, 25.271, and 26.849; and most preferably, the X-ray powder diffraction pattern expressed with diffraction angle 2*θ* is as shown in FIG. 3.
4. Crystal form B2 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 8.182, 8.839, 10.401, 11.168, and 18.906; preferably characteristic peaks at 8.182, 8.839, 10.401, 11.168, 11.679, 13.714, 18.906, 20.245, 21.895, and 25.134; more preferably characteristic peaks at 8.182, 8.839, 10.401, 11.168, 11.679, 13.714, 14.880, 16.592, 17.660, 18.906, 20.245, 21.895, 22.600, and 25.134; and most preferably, the X-ray powder diffraction pattern expressed with diffraction angle 2*θ* is as shown in FIG. 4.
5. Crystal form B3 of compound 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid, wherein an X-ray powder diffraction pattern expressed with diffraction angle 2*θ* has characteristic peaks at 10.548, 11.496, 17.557, 19.135, 19.751, and 25.360; preferably characteristic peaks at 10.548, 11.269, 11.496, 17.557, 18.103, 19.135, 19.751, 20.605, 22.767, and 25.360; more preferably characteristic peaks at 8.224, 8.976, 10.548, 11.269, 11.496, 12.264, 13.730, 14.829, 17.557, 18.103, 19.135, 19.751, 20.605, 22.767, 23.522, 24.738, 25.360, 26.556, and 26.893; and most preferably, the X-ray powder diffraction pattern expressed with diffraction angle 2*θ* is as shown in FIG. 5.
6. Crystal form C of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 10.094, 11.511, 17.378, and 20.113; preferably characteristic peaks at 10.094, 11.511, 15.875, 17.378, 17.763, 18.573, 20.113, and 22.925; more preferably characteristic peaks at 5.470, 10.094, 11.511, 12.138, 14.975, 15.875, 17.378, 17.763, 18.573, 19.413, 20.113, 22.925, 23.881, 26.177, and 28.163; and most preferably, the X-ray powder diffraction pattern expressed with diffraction angle 2*θ* is as shown in FIG. 6.
7. Crystal form D of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 10.940, 12.216, 18.344, 19.931, and 22.979; preferably characteristic peaks at 6.343, 10.940, 12.216, 17.695, 18.344, 18.973, 19.472, 19.931, 21.753, 22.979, and 24.685; more preferably characteristic peaks at 6.343, 10.940, 12.216, 12.762, 14.684, 16.167, 16.510, 17.695, 18.344, 18.973, 19.472, 19.931, 21.753, 22.979, 24.306, 24.685, and 25.898; and most preferably, the X-ray powder diffraction pattern expressed with diffraction angle 2*θ* is as shown in FIG. 7.
8. Crystal form E of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 11.591, 17.645, 19.060, 20.066, 20.667, and 26.987; preferably characteristic peaks at 9.261, 10.735, 11.591, 13.946, 17.645, 18.291, 19.060, 20.066, 20.667, and 26.987; more preferably characteristic peaks at 8.245, 8.738, 9.261, 10.735, 11.591, 12.056, 13.946, 14.925, 16.922, 17.645, 18.291, 19.060, 20.066, 20.667, 22.474, 24.608, and 26.987; and most preferably, the X-ray powder diffraction pattern expressed with diffraction angle 2*θ* is as shown in FIG. 8.
9. Crystal form F of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 9.543, 19.405, and 22.153; preferably characteristic peaks at 9.543, 11.421, 14.557, 16.175, 17.886, 19.405, 22.153, and 25.821; and more preferably, the X-ray powder diffraction pattern expressed with diffraction angle 2*θ* is as shown in FIG. 9.
10. Crystal form G of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid, wherein an X-ray powder diffraction pattern expressed with diffraction angle 2*θ* has characteristic peaks at 9.096, 11.107, 17.239, and 17.744; preferably characteristic peaks at 6.120, 9.096, 11.107, 12.302, 13.387, 17.239, 17.744, 22.984, 23.981, and 25.879; more preferably characteristic peaks at 6.120, 9.096, 9.519, 11.107, 12.302, 13.387, 14.833, 17.239, 17.744, 20.302, 20.905, 22.416, 22.984, 23.342, 23.981, 25.879, and 28.791; and most preferably, the X-ray powder diffraction pattern expressed with diffraction angle 2*θ* is as shown in FIG. 10.
11. The crystal form according to any one of aspects 1 to 10, wherein the error range of the 2*θ* value is ± 0.2.
12. A method for preparing the crystal form A, B1, B2, B3, C, D, E, F, or G according to any one of aspects 1 to 11, selected from any one of the following methods:
   method I:
      (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent, and dissolving the mixture by means of stirring or heating,
      (b) crystallization;
   or method II:
      (a) mixing compound 2-((4-((*S*)-3-(4-chloro-2-fluorophenyl)-2,3-dihydrobenzo [*b*][1,4]dioxan-5-yl)piperidin-1-yl)methyl)-1-(((*S*)-oxetan-2-yl)methyl)-1*H*-benzo[d]imidazole-6-carboxylic acid with a solvent, and dissolving the mixture by means of stirring or heating,
      (b) adding a second solvent for crystallization;
   or method III:
      (a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H-*benzo[*d*]imidazole-6-carboxylic acid with a solvent,
      (b) stirring and pulping.
13. A pharmaceutical composition comprising the crystal form according to any one of aspects 1 to 11 or a crystal form prepared by the method according to aspect 12, and optionally a pharmaceutically acceptable excipient.
14. A method for preparing a pharmaceutical composition, the method comprising a step of mixing the crystal form according to any one of aspects 1 to 11 or a crystal form prepared by the method according to aspect 12 with a pharmaceutically acceptable excipient.
15. Use of the crystal form according to any one of aspects 1 to 11 or a crystal form prepared by the method according to aspect 12 or the composition according to aspect 13 in the preparation of a medicament for treating or preventing a disease associated with GLP-1 receptor.
16. Use of the crystal form according to any one of aspects 1 to 11 or a crystal form prepared by the method according to aspect 12 or the composition according to aspect 13 in the preparation of a medicament for treating or preventing diabetes.

## Claims

1. Crystal form B1 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 8.135, 8.915, 11.259, 11.508, 19.024, and 25.271; preferably characteristic peaks at 8.135, 8.915, 10.507, 11.259, 11.508, 12.223, 16.751, 19.024, 22.736, and 25.271; more preferably characteristic peaks at 8.135, 8.915, 10.507, 11.259, 11.508, 12.223, 13.632, 15.055, 16.751, 17.836, 19.024, 20.541, 22.205, 22.736, 25.271, and 26.849; and most preferably, the X-ray powder diffraction pattern expressed with diffraction angle 2*θ* is as shown in FIG. 3,
wherein the error range of the 2*θ* value is ± 0.2.

2. Crystal form B2 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 8.182, 8.839, 10.401, 11.168, and 18.906; preferably characteristic peaks at 8.182, 8.839, 10.401, 11.168, 11.679, 13.714, 18.906, 20.245, 21.895, and 25.134; more preferably characteristic peaks at 8.182, 8.839, 10.401, 11.168, 11.679, 13.714, 14.880, 16.592, 17.660, 18.906, 20.245, 21.895, 22.600, and 25.134; and most preferably, the X-ray powder diffraction pattern expressed with diffraction angle 2*θ* is as shown in FIG. 4,
wherein the error range of the 2*θ* value is ± 0.2.

3. Crystal form B3 of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 10.548, 11.496, 17.557, 19.135, 19.751, and 25.360; preferably characteristic peaks at 10.548, 11.269, 11.496, 17.557, 18.103, 19.135, 19.751, 20.605, 22.767, and 25.360; more preferably characteristic peaks at 8.224, 8.976, 10.548, 11.269, 11.496, 12.264, 13.730, 14.829, 17.557, 18.103, 19.135, 19.751, 20.605, 22.767, 23.522, 24.738, 25.360, 26.556, and 26.893; and most preferably, the X-ray powder diffraction pattern expressed with diffraction angle 2*θ* is as shown in FIG. 5,
wherein the error range of the 2*θ* value is ± 0.2.

4. Crystal form C of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 10.094, 11.511, 17.378, and 20.113; preferably characteristic peaks at 10.094, 11.511, 15.875, 17.378, 17.763, 18.573, 20.113, and 22.925; more preferably characteristic peaks at 5.470, 10.094, 11.511, 12.138, 14.975, 15.875, 17.378, 17.763, 18.573, 19.413, 20.113, 22.925, 23.881, 26.177, and 28.163; and most preferably, the X-ray powder diffraction pattern expressed with diffraction angle 2*θ* is as shown in FIG. 6,
wherein the error range of the 2*θ* value is ± 0.2.

5. Crystal form D of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 10.940, 12.216, 18.344, 19.931, and 22.979; preferably characteristic peaks at 6.343, 10.940, 12.216, 17.695, 18.344, 18.973, 19.472, 19.931, 21.753, 22.979, and 24.685; more preferably characteristic peaks at 6.343, 10.940, 12.216, 12.762, 14.684, 16.167, 16.510, 17.695, 18.344, 18.973, 19.472, 19.931, 21.753, 22.979, 24.306, 24.685, and 25.898; and most preferably, the X-ray powder diffraction pattern expressed with diffraction angle 2*θ* is as shown in FIG. 7,
wherein the error range of the 2*θ* value is ± 0.2.

6. Crystal form E of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 11.591, 17.645, 19.060, 20.066, 20.667, and 26.987; preferably characteristic peaks at 9.261, 10.735, 11.591, 13.946, 17.645, 18.291, 19.060, 20.066, 20.667, and 26.987; more preferably characteristic peaks at 8.245, 8.738, 9.261, 10.735, 11.591, 12.056, 13.946, 14.925, 16.922, 17.645, 18.291, 19.060, 20.066, 20.667, 22.474, 24.608, and 26.987; and most preferably, the X-ray powder diffraction pattern expressed with diffraction angle 2*θ* is as shown in FIG. 8,
wherein the error range of the 2*θ* value is ± 0.2.

7. Crystal form F of compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid, wherein an X-ray powder diffraction pattern, obtained using Cu-Kα radiation (λ=1.5406 Å) and expressed with diffraction angle 2*θ*, has characteristic peaks at 9.543, 19.405, and 22.153; preferably characteristic peaks at 9.543, 11.421, 14.557, 16.175, 17.886, 19.405, 22.153, and 25.821; and more preferably, the X-ray powder diffraction pattern expressed with diffraction angle 2*θ* is as shown in FIG. 9,
wherein the error range of the 2*θ* value is ± 0.2.

8. A method for preparing the crystal form B1 according to claim 1, selected from any one of the following methods:
method I:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, and dissolving the mixture by means of stirring or heating, wherein the solvent is selected from one or more of isopropanol, isopentanol, 1,2-dichloroethane, acetone, isopropyl acetate, propylene glycol methyl ether, and p-xylene, and
(b) crystallization;
or method II:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent, and dissolving the mixture by means of stirring or heating, wherein the solvent is tetrahydrofuran, and
(b) adding a solvent for crystallization, wherein the solvent is water;
or method III:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*-benzo[*d*]imidazole-6-carboxylic acid with a solvent, wherein the solvent is dioxane, and
(b) stirring and pulping.

9. A method for preparing the crystal form B2 according to claim 2, comprising:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, and dissolving the mixture by means of stirring or heating, wherein the solvent is methyl isobutyl ketone, and
(b) crystallization.

10. A method for preparing the crystal form B3 according to claim 3, comprising:
(a) mixing compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, and dissolving the mixture by means of stirring or heating, wherein the solvent is selected from one or more of ethyl acetate, dichloromethane, and 10% water/acetone, and
(b) crystallization.

11. A method for preparing the crystal form C according to claim 4, selected from any one of the following methods:
method (I):
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, and dissolving the mixture by means of stirring or heating, wherein the solvent is toluene, and
(b) crystallization;
or method (II):
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, wherein the solvent is isopropyl acetate, and
(b) stirring and pulping.

12. A method for preparing the crystal form D according to claim 5, selected from any one of the following methods:
method (I):
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, and dissolving the mixture by means of stirring or heating, wherein the solvent is selected from 50% methanol/water and 50% acetonitrile/methanol, and
(b) crystallization;
or method II:
(a) mixing compound 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1Hbenzo[d]imidazole-6-carboxylic acid with a solvent, and dissolving the mixture by means of stirring or heating, wherein the solvent is selected from tetrahydrofuran and chloroform, and
(b) adding a solvent for crystallization, wherein the solvent is methanol;
or method III:
(a) mixing compound 2-((4-((*S*)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1*H*benzo[*d*]imidazole-6-carboxylic acid with a solvent, wherein the solvent is methanol, and
(b) stirring and pulping.

13. A method for preparing the crystal form E according to claim 6, selected from any one of the following methods:
method (I):
(a) mixing compound 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1Hbenzo[d]imidazole-6-carboxylic acid with a solvent, and dissolving the mixture by means of stirring or heating, wherein the solvent is acetonitrile, and
(b) crystallization;
or method II:
(a) mixing compound 2-((4-((S)-3-(4-chloro-2-fluorophenyl)-2,3-dihydrobenzo[*b*][1,4]dioxan-5-yl)piperidin-1-yl)methyl)-1-(((*S*)-oxetan-2-yl)methyl)-1Hbenzo[d]imidazole-6-carboxylic acid with a solvent, and dissolving the mixture by means of stirring or heating, wherein the solvent is tetrahydrofuran, and
(b) adding a solvent for crystallization, wherein the solvent is acetonitrile.

14. A method for preparing the crystal form F according to claim 7, selected from any one of the following methods:
method (I):
(a) mixing compound 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1Hbenzo[d]imidazole-6-carboxylic acid with a solvent, and dissolving the mixture by means of stirring or heating, wherein the solvent is dimethyl sulfoxide, and
(b) crystallization;
or method II:
(a) mixing compound 2-((4-((S)-3-(4-chloro-2-fluorophenyl)-2,3-dihydrobenzo[*b*][1,4]dioxan-5-yl)piperidin-1-yl)ymethyl)-1-(((*S*)-oxetan-2-yl)methyl)-1Hbenzo[d]imidazole-6-carboxylic acid with a solvent, and dissolving the mixture by means of stirring or heating, wherein the solvent is dimethyl sulfoxide, and
(b) adding a solvent for crystallization, wherein the solvent is water.

15. A pharmaceutical composition comprising the crystal form according to any one of claims 1-7, and optionally a pharmaceutically acceptable excipient.

16. A method for preparing a pharmaceutical composition, the method comprising a step of mixing the crystal form according to any one of claims 1-7 with a pharmaceutically acceptable excipient.

17. The crystal form according to claim 1-7, or the composition according to claim 15 for use in the treatment or prevention of diabetes.
